# EUROPEAN PATENT SPECIFICATION

(11) **EP 0 562 576 B1**
(45) Date of publication and mention of the grant of the patent: **22.01.2003**
(21) Application number: 93104870.6
(22) Date of filing: 24.03.1993
(51) Int. Cl.: C07D 251/46

(54) **Process for the preparation of phenyl (1,3,5-triazin-2-yl) carbamates**
Verfahren zur Herstellung von Phenyl (1,3,5-Triazin-2-Yl) Carbamaten
Procédé pour la préparation de phényl (1,3,5-triazin-2-yl) carbamates

(30) Priority: 26.03.1992 JP 9859792; 02.06.1992 JP 16553092
(43) Date of publication of application: 29.09.1993
(60) Divisional application: 02003230.6
(73) Proprietor: KUREHA KAGAKU KOGYO KABUSHIKI KAISHA, Chuo-ku, Tokyo 103-8552 (JP)
(72) Inventor: Kanda, Yoichi, Iwaki-shi, Fukushima-ken 974 (JP); Arabori, Hideo, Iwaki-shi, Fukushima-ken 974 (JP)
(74) Representative: Kraus, Walter, Dr.

(56) References cited:
- EP-A- 0 103 543
- EP-A- 0 205 323
- EP-A- 0 238 070

## Description

### BACKGROUND OF THE INVENTION

This invention relates to a process for the preparation of phenyl (1,3,5-triazin-2-yl)carbamates which are useful intermediates for producing of herbicidally active sulfonylurea compounds.

EP-A-85028(corresponding to JP-A-58146572), EP-A-103543(corresponding to JP-A-5973583), JP-A-60139691 and EP-A-238070(corresponding to JP-A-6438091) disclose that phenyl (1,3,5-triazin-2-yl)carbamates and phenyl (pyrimidin-2-yl)carbamates are used as intermediates for producing of herbicidally active sulfonylurea compounds.

The preparations of phenyl (pyrimidin-2-yl)carbamates are relatively simple methods and have a good yield.

However, a simple synthesis method of phenyl (1,3,5-triazin-2-yl)carbamates is not reported. (c.f. reference examples 53 and 57 in EP-A-238070)

### SUMMARY OF THE INVENTION

An object of the present invention is to provide a process to prepare phenyl (1,3,5-triazin-2-yl)carbamates of the formula (I) as shown below, which is simple and has a good yield: 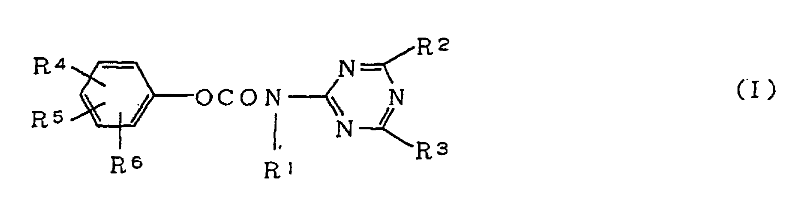 wherein
R¹ is hydrogen atom, C₁-C₄ alkyl or C₁-C₄ alkoxy
R² and R³ are independently a hydrogen atom, halogen atom, C₁-C₄ alkyl, C₁-C₄ alkoxy, C₁-C₄ alkylthio, C₁-C₄ haloalkyl, C₁-C₄ haloalkoxy, C₁-C₄ haloalkylthio, C₂-C₄ alkoxyalkyl or NR⁷R⁸
R⁷ and R⁸ are independently a hydrogen atom or C₁-C₄ alkyl
R⁴, R⁵ and R⁶ are independently a hydrogen atom, halogen atom, C₁-C₄ alkyl or C₁-C₄ alkoxy

### DETAILED DESCRIPTION OF THE INVENTION

According to the present invention there is therefore provided a process for the preparation of a phenyl (1,3,5-triazin 2-yl)carbamate of the formula (I) 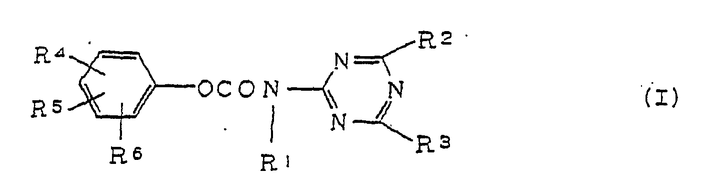 wherein
R¹ is a hydrogen atom, C₁-C₄ alkyl or C₁-C₄ alkoxy,
R² and R³ are independently a hydrogen atom, halogen atom, C₁-C₄ alkyl, C₁-C₄ alkoxy, C₁-C₄ alkylthio, C₁-C₄ haloalkyl, C₁-C₄ haloalkoxy, C₁-C₄ haloalkylthio, C₂-C₄ alkoxyalkyl or NR⁷R⁸,
R⁷ and R⁸ are independently a hydrogen atom or C₁-C₄ alkyl,
R⁴, R⁵ and R⁶ are independently a hydrogen atom, halogen atom, C₁-C₄ alkyl or C₁-C₄ alkoxy,
which process comprises :
reacting a 2-amino-1,3,5-triazine of the formula (II) 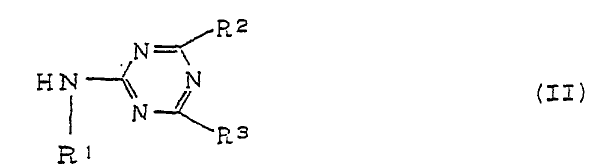 in an aprotic polar solvent, with a diphenyl carbonate of the formula (III) 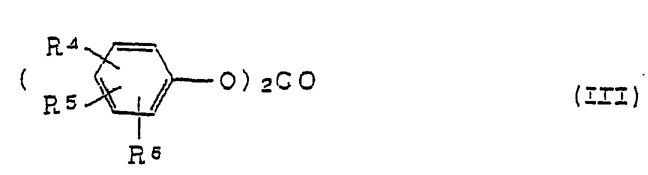
characterized in that
(i) said 2-amino-1,3,5-triazine is reacted with an alkali metal hydride
(ii) said aprotic polar solvent is N,N-dimethylacetamide or N-methylpyrrolidone, and
(iii) the addition of the diphenyl carbonate of the formula (III) is effected after evolution of hydrogen gas in step (i),
or characterized in that
(vi) said diphenyl carbonate is reacted with an alkali metal salt of said 2-amino-1,3,5-triazine, and
(v) said aprotic polar solvent is N,N-dimethylacetamide or N-methylpyrrolidone.

Halogen in the above definitions, and also as a moiety of haloalkyl, haloalkoxy and haloalkylthio, is fluorine, chlorine, bromine and iodine, with fluorine and chlorine being preferred.

Haloalkyl, haloalkoxy and haloalkylthio are substituted with one or more halogen atoms.

In suitable 2-amino-1,3,5-triazines of the formula (II), R¹ is a hydrogen atom, R² and R³ are independently C₁-C₂ alkyl, C₁-C₂ alkoxy, C₁-C₂ fluoroalkyl, C₁-C₂ fluoroalkoxy, C₂-C₄ alkoxyalkyl or NR⁷R⁸, R⁷ and R⁸ are independently a hydrogen atom or C₁-C₂ alkyl.

Fluoroalkyl, fluoroalkoxy and fluoroalkylthio in the above definitions are substituted with one or more fluorine atoms.

In suitable diphenyl carbonates of the formula (III), R⁴, R⁵ and R⁶ are independently a hydrogen atom or methyl.

The process of the invention is illustrated by the following reaction scheme, 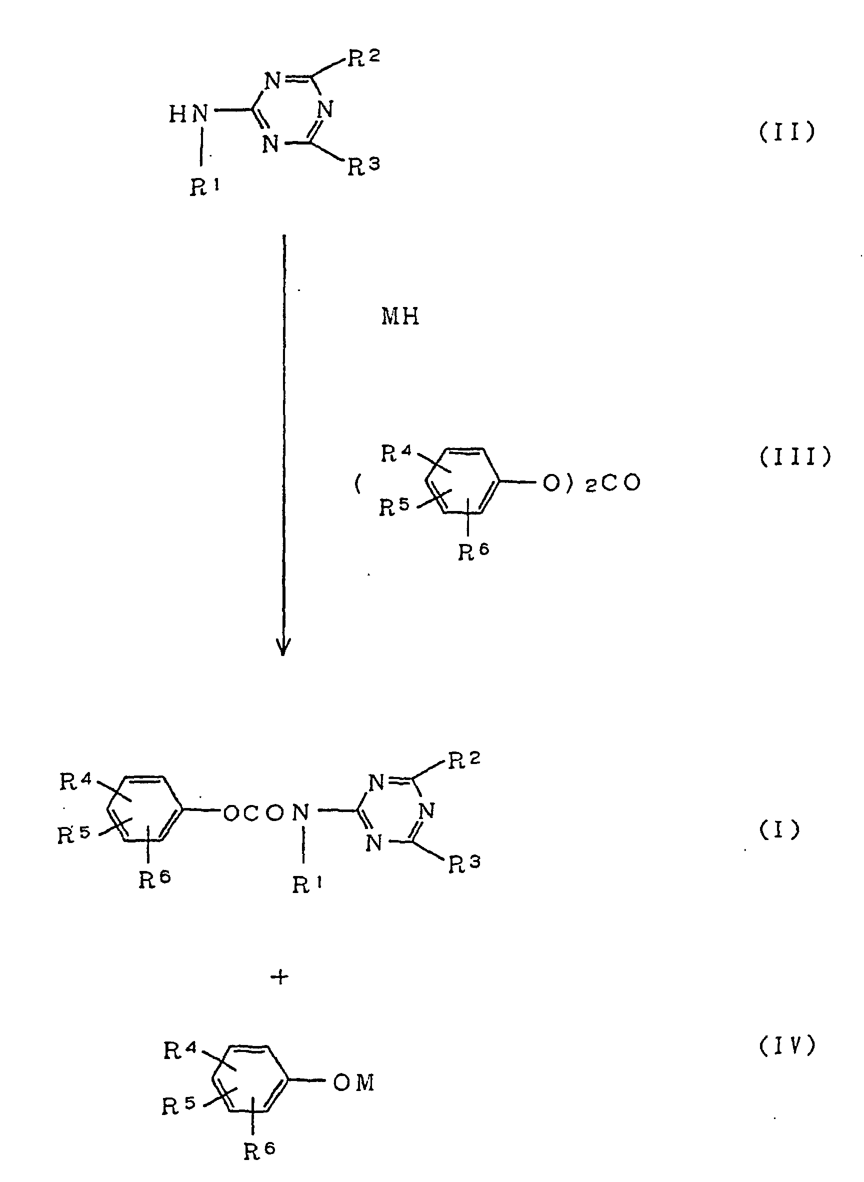 wherein R¹, R², R³, R⁴, R⁵ and R⁶ are as defined above and M is an alkali metal atom.

As suitable alkali metal hydrides, sodium hydride or potassium hydride may be used.

The reaction is preferably carried out at a temperature from 0 to 40 °C to give phenyl (1,3,5-triazin-2-yl)carbamates of the formula (I) in good yields.

The reaction temperature may be out of the range from 0 to 40 °C, if necessary and if it does not give the product in bad yields.

The reaction time is preferably 5-30 minutes after mixing 2-amino-1,3,5-triazines of the formula (II), alkali metal hydrides and diphenyl carbonates of the formula (III).

But, elongation of the reaction time is allowed if it does not affect the reaction yield.

Alkali metal hydrides and diphenyl carbonates of the formula (III) are employed an equimolar amount per mole of 2-amino-1,3,5-triazines of the formula (II), respectively, to give the objective compound in good yields.

But, per mole of the most expensive starting compound, excesses of the other starting compounds may be used if this does not affect the reaction yield.

The used amount of a reaction solvent is not restricted especially, since the object of the invention is achieved once the starting materials are mixed well in it.

The ratio of the sum of used starting materials (gr. weight) to used solvent (ml. volume) is preferably in the range of 1/3 - 1/10 (gr/ml).

In the case of isolating phenyl (1,3,5-triazine-2-yl)carbamates of the formula (I) from the resultant mixture of above reaction operation, the resultant mixture is poured into the iced-water containing 1.1 - 5 moles of hydrogen chloride per mole of used alkali metal hydride.

The precipitate is filtered off and washed with petroleum ether or hexane to give the product.

Of course, if necessary, recrystallization or column chromatography may be used for further purification process.

However, in the case of using phenyl (1,3,5-triazin-2-yl)carbamates of the formula (I) for the reagent of [(1,3,5-triazin-2-yl)amino]carbonylation at the nitrogen atom of a sulfonamide group, the aprotic polar solvent containing phenyl (1,3,5-triazin-2-yl)carbamates of the formula (I) and alkali metal phenoxides of the formula (IV) (corresponding to the above resultant reaction mixture) may be used without further adding base compounds.

Therefore, the use of the above resultant reaction mixture is of commercial interest on account of the advantages mentioned.

The invention is further illustrated by the following examples, in which 2-amino-4,6-dimethoxy-1,3,5-triazine as 2-amino-1,3,5-triazines of the formula (II), diphenyl carbonate as diphenyl carbonates of the formula (III), sodium hydride as alkali metal hydrides, N,N-dimethylacetamide as aprotic polar solvents and methyl 2-(aminosulfonyl)benzoate as compounds having a sulfonamide group are used.

### Example 1

### The preparation of phenyl (4,6-dimethoxy-1,3,5-triazin-2-yl)carbamate

Into 50 ml Erlenmeyer flask, were added 2-amino-4,6-dimethoxy-1,3,5-triazine 2.00 g (0.0128 mole) and dry N,N-dimethylacetamide 18 ml and stirred in the suspended condition.

To the suspended mixture, at the room temperature, was added 60 % oily sodium hydride 0.51 g (0.0128 mole) (equal to the preparation of sodium salt of 2-amino-4,6-dimethoxy-1,3,5-triazine).

The reaction mixture was homogeneous after vigorous evolution of hydrogen gas.

A solution of diphenyl carbonate 2.74 g (0.0128 mole) and dry N,N-dimethylacetamide was subsequently added to the mixture under the water cooling during 10 minutes, and stirred for further 10 minutes.

The reaction mixture was poured into 20 ml iced water containing 1.4 ml hydrogen chloride (35 % aq soln).

The precipitate was filtered off and wash with iced water and then petroleum ether.

After being air-dried, the product was obtained.

The obtained amount (yield of theory) and the physical property were showed hereinafter.
2.60 g (73.5 % of theory)
mp.141-142 °C
IR(KBr,cm⁻¹) 3304, 1780, 1752, 1618, 1482 1390, 1360, 1290, 1192
NMR(CDCl₃, δ) 4.0(6H,s,OCH₃ × 2 ) 7.05 - 7.51(5H, m, Aromatic H) 8.28 - 8.7(1H,bs,NH)

### Example 2

The use of the N,N-dimethylacetamide mixture for [(1,3,5-triazin-2-yl)amino]carbonylation at the nitrogen atom of a sulfonamide group

### [1]

### Preparation of the N,N-dimethylacetamide mixture containing phenyl (4,6-dimethoxy-1,3,5-triazin-2-yl)carbamate and sodium phenoxide

Into 50 ml Erlenmeyer flask, were added 2-amino-4,6-dimethoxy-1,3,5-triazine 1.00 g and dry N,N-dimethylacetamide 9 ml, and stirred in suspended condition.

To the suspended mixture, at the room temperature, was added 60 % oily sodium hydride 0.26 g (equal to the preparation of sodium salt of 2-amino-4,6-dimethoxy-1,3,5-triazine).

The reaction mixture was homogeneous after vigorous evolution of hydrogen gas.

A solution of diphenyl carbonate 1.37 g and dry N,N-dimethylacetamide 4.2 ml was subsequently added dropwise to the mixture under water cooling during 6 minutes.

Stirring was maintained for further 10 minutes to prepare the N,N-dimethylacetamide mixture containing phenyl (4,6-dimethoxy-1,3,5-triazin-2-yl)carbamate and sodium phenoxide.

### [2]

### Preparation of methyl 2-[[(4,6-dimethoxy-1,3,5-triazin-2-yl)aminocarbonyl]aminosulfonyl]benzoate

The N,N-dimethylacetamide mixture in 50 ml Erlenmeyer flask, obtained in [1], was stirred under iced water cooling.

To the mixture, was added dropwise a solution of methyl 2-(aminosulfonyl)benzoate 1.25 g and N,N-dimethylacetamide 3 ml for 5 minutes.

The mixture was stirred for further 1.5 hours.

The mixture was subsequently poured into 130 ml iced water containing 0.7 ml hydrogen chloride (35 % aq soln).

A white precipitate was filtered off and washed with iced water and then petroleum ether, and air-dried to give the product.

The obtained amount (yield of theory) and the physical property were showed hereinafter.
1.90 g (82.2 % of theory)
purity by HPLC 97.8 %
mp.171-173 °C
IR (KBr,cm⁻¹) 3320, 1742, 1608, 1494, 1388, 1292
NMR (d₆-DMSO , δ) 3.8(3H, s) 3.95(6H, s) 7.26 - 8.3 (5H, m) 10.9(1H, bs)

## Claims

1. A process for the preparation of a phenyl (1,3,5-triazin 2-yl)carbamate of the formula (I) 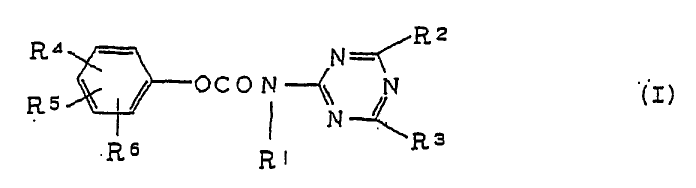 wherein
R¹ is a hydrogen atom, C₁-C₄ alkyl or C₁-C₄ alkoxy,
R² and R³ are independently a hydrogen atom, halogen atom, C₁-C₄ alkyl, C₁-C₄ alkoxy, C₁-C₄ alkylthio, C₁-C₄ haloalkyl, C₁-C₄ haloalkoxy, C₁-C₄ haloalkylthio, C₂-C₄ alkoxyalkyl or NR⁷R⁸,
R⁷ and R⁸ are independently a hydrogen atom or C₁-C₄ alkyl,
R⁴, R⁵ and R⁶ are independently a hydrogen atom, halogen atom, C₁-C₄ alkyl or C₁-C₄ alkoxy,
which process comprises:
reacting a 2-amino-1,3,5-triazine of the formula (II) 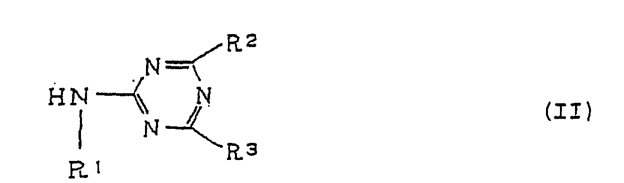 in an aprotic polar solvent, with a diphenyl carbonate of the formula (III) 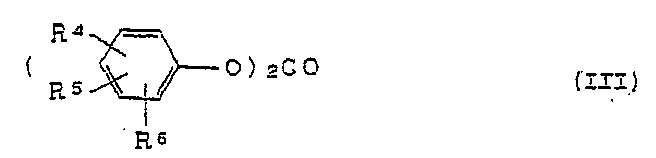
**characterized in that**
(i) said 2-amino-1,3,5-triazine is reacted with an alkali metal hydride
(ii) said aprotic polar solvent is N,N-dimethylacetamide or N-methylpyrrolidone, and
(iii) the addition of the diphenyl carbonate of the formula (III) is effected after evolution of hydrogen gas in step (i).

2. A process for the preparation of a phenyl (1,3,5-triazin 2-yl)carbamate of the formula (I) 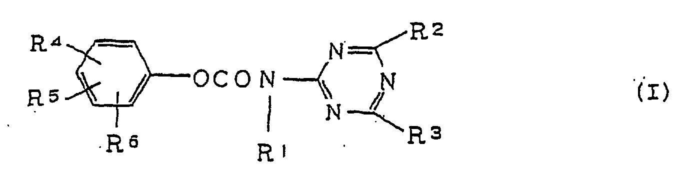 wherein
R¹ is a hydrogen atom, C₁-C₄ alkyl or C₁-C₄ alkoxy,
R² and R³ are independently a hydrogen atom, halogen atom, C₁-C₄ alkyl, C₁-C₄ alkoxy, C₁-C₄ alkylthio, C₁-C₄ haloalkyl, C₁-C₄ haloalkoxy, C₁-C₄ haloalkylthio, C₂-C₄ alkoxyalkyl or NR⁷R⁸,
R⁷ and R⁸ are independently a hydrogen atom or C₁-C₄ alkyl,
R⁴, R⁵ and R⁶ are independently a hydrogen atom, halogen atom, C₁-C₄ alkyl or C₁-C₄ alkoxy;
which process comprises:
reacting a 2-amino-1,3,5-triazine of the formula (II) 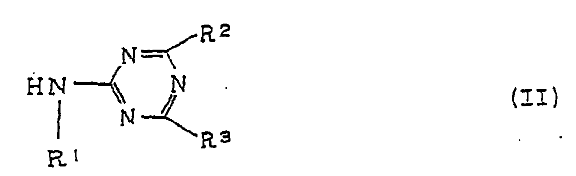 in an aprotic polar solvent, with a diphenyl carbonate of the formula (III) 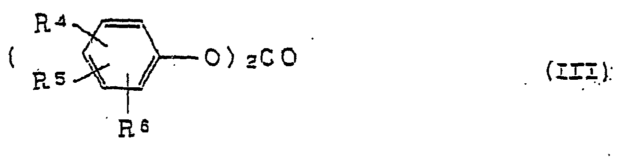
**characterized in that**
(vi) said diphenyl carbonate is reacted with an alkali metal salt of said 2-amino-1,3,5-triazine, and
(v) said aprotic polar solvent is N,N-dimethylacetamide or N-methylpyrrolidone.

## Patentansprüche

1. Verfahren zur Herstellung von Phenyl-(1,3,5-triazin-2-yl)carbamat der Formel (I) 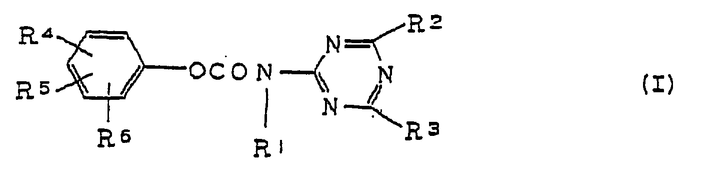 worin
R¹ ein Wasserstoffatom, C₁-C₄-Alkyl oder C₁-C₄-Alkoxy bedeutet,
R² und R³ unabhängig ein Wasserstoffatom, ein Halogenatom, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, C₂-C₄-Alkoxyalkyl oder NR⁷R⁸ bedeuten,
R⁷ und R⁸ unabhängig ein Wasserstoffatom oder C₁-C₄-Alkyl bedeuten,
R⁴, R⁵ und R⁶ unabhängig ein Wasserstoffatom, ein Halogenatom, C₁-C₄-Alkyl oder C₁-C₄-Alkoxy bedeuten,
umfassend:
die Umsetzung eines 2-Amino-1,3,5-triazins der Formel (II) 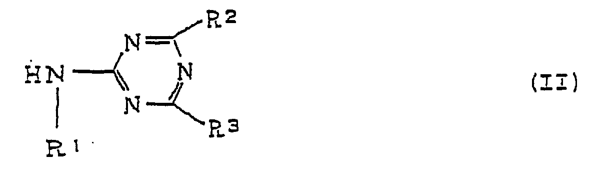
in einem aprotischen polaren Lösungsmittel mit einem Diphenylcarbonat der Formel (III) 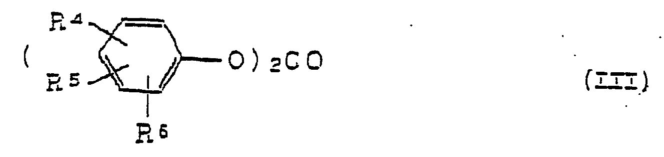
**dadurch gekennzeichnet, dass**
(i) das 2-Amino-1,3,5-triazin mit einem Alkalimetallhydrid umgesetzt wird
(ii) das aprotische polare Lösungsmittel N,N-Dimethylacetamid oder N-Methylpyrrolidon ist, und
(iii) die Zugabe von Diphenylcarbonat der Formel (III) unter Bildung von Wasserstoffgas bei Stufe (i) erfolgt.

2. Verfahren zur Herstellung von Phenyl-(1,3,5-triazin-2-yl)carbamat der Formel (I) 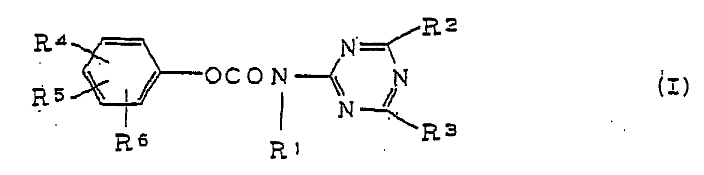 worin
R¹ ein Wasserstoffatom, C₁-C₄-Alkyl oder C₁-C₄-Alkoxy bedeutet,
R² und R³ unabhängig ein Wasserstoffatom, ein Halogenatom, C₁-C₄-Alkyl, C₁-C₄-Alkoxy, C₁-C₄-Alkylthio, C₁-C₄-Halogenalkyl, C₁-C₄-Halogenalkoxy, C₁-C₄-Halogenalkylthio, C₂-C₄-Alkoxyalkyl oder NR⁷R⁸ bedeuten,
R⁷ und R⁸ unabhängig ein Wasserstoffatom oder C₁-C₄-Alkyl bedeuten,
R⁴, R⁵ und R⁶ unabhängig ein Wasserstoffatom, ein Halogenatom, C₁-C₄-Alkyl oder C₁-C₄-Alkoxy bedeuten,
umfassend:
die Umsetzung eines 2-Amino-1,3,5-triazins der Formel (II) 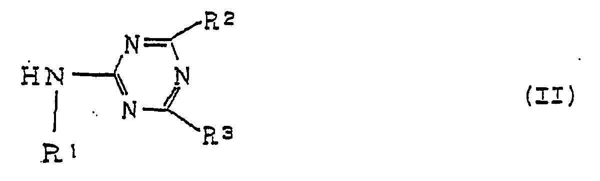
in einem aprotischen polaren Lösungsmittel mit einem Diphenylcarbonat der Formel (III) 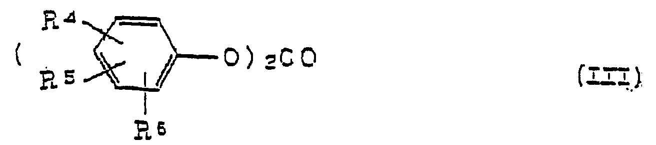
**dadurch gekennzeichnet, dass**
(vi) das Diphenylcarbonat mit einem Alkalimetallsalz des 2-Amino-1,3,5-triazins umgesetzt wird und
(v) das aprotische polare Lösungsmittel N,N-Dimethylacetamid oder N-Methylpyrrolidon ist.

## Revendications

1. Procédé pour la préparation d'un (1,3,5-triazin-2-yl)-carbamate de phényle de formule (I) : 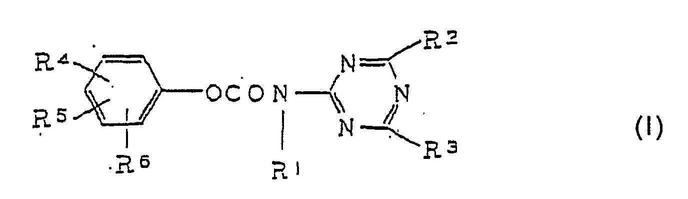 dans laquelle :
R¹ est un atome d'hydrogène, un groupe alkyle en C₁₋₄ ou alcoxy en C₁₋₄,
R² et R³ sont indépendamment un atome d'hydrogène, un atome d'halogène, un groupe alkyle en C₁₋₄, alcoxy en C₁₋₄, alkylthio en C₁₋₄, haloalkyle en C₁₋₄, haloalcoxy en C₁₋₄, haloalkylthio en C₁₋₄, alcoxyalkyle en C₂₋₄ ou NR⁷R⁸,
R⁷ et R⁸ sont indépendamment un atome d'hydrogène ou un groupe alkyle en C₁₋₄,
R⁴, R⁵ et R⁶ sont indépendamment un atome d'hydrogène, un atome d'halogène, un groupe alkyle en C₁₋₄ ou alcoxy en C₁₋₄,
lequel procédé comprend :
la réaction d'une 2-amino-1,3,5-triazine de formule (II) : 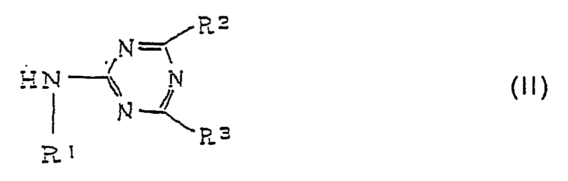
dans un solvant polaire aprotique,
avec un carbonate de diphényle de formule (III) : 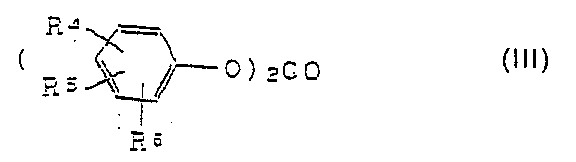
**caractérisé en ce que** :
(i) ladite 2-amino-1,3,5-triazine est traitée avec un hydrure de métal alcalin,
(ii) ledit solvant polaire aprotique est le N,N-diméthylacétamide ou la N-méthylpyrrolidone, et
(iii) l'addition du carbonate de diphényle de formule (III) est effectuée après le dégagement d'hydrogène gazeux dans l'étape (i).

2. Procédé pour la préparation d'un (1,3,5-triazin-2-yl)-carbamate de phényle de formule (I) : 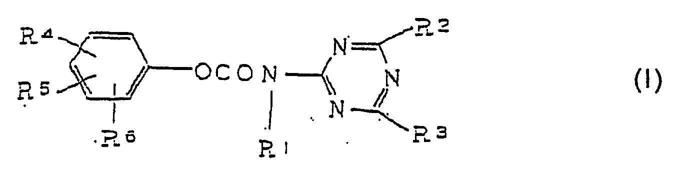 dans laquelle :
R¹ est un atome d'hydrogène, un groupe alkyle en C₁₋₄ ou alcoxy en C₁₋₄,
R² et R³ sont indépendamment un atome d'hydrogène, un atome d'halogène, un groupe alkyle en C₁₋₄, alcoxy en C₁₋₄, alkylthio en C₁₋₄, haloalkyle en C₁₋₄, haloalcoxy en C₁₋₄, haloalkylthio en C₁₋₄, alcoxyalkyle en C₂₋₄ ou NR⁷R⁸,
R⁷ et R⁸ sont indépendamment un atome d'hydrogène ou un groupe alkyle en C₁₋₄,
R⁴, R⁵ et R⁶ sont indépendamment un atome d'hydrogène, un atome d'halogène, un groupe alkyle en C₁₋₄ ou alcoxy en C₁₋₄,
lequel procédé comprend
la réaction d'une 2-amino-1,3,5-triazine de formule (II) : 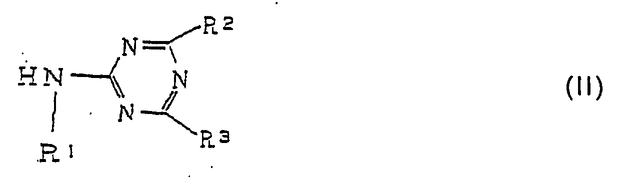 dans un solvant polaire aprotique,
avec un carbonate de diphényle de formule (III) : 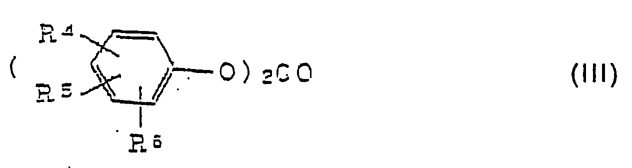
**caractérisé en ce que** :
(iv) ledit carbonate de diphényle est traité avec un sel de métal alcalin de ladite 2-amino-1,3,5-triazine, et
(v) ledit solvant polaire aprotique est le N,N-diméthylacétamide ou la N-méthylpyrrolidone.
